# EUROPÄISCHE PATENTSCHRIFT

(11) **EP 4 308 529 B1**
(45) Veröffentlichungstag und Bekanntmachung des Hinweises auf die Patenterteilung: **06.05.2026**
(21) Anmeldenummer: 22714818.6
(22) Anmeldetag: 14.03.2022
(51) Int. Cl.: C07C 5/48, C07C 11/04, C07C 51/215, C07C 53/08, B01J 8/06, B01J 23/20

(54) **VERFAHREN ZUR HERSTELLUNG EINER ZIELVERBINDUNG**
PROCESS FOR PRODUCING A TARGET COMPOUND
PROCÉDÉ DE PRODUCTION D'UN COMPOSÉ CIBLE

(30) Priorität: 15.03.2021 DE 102021202495
(43) Veröffentlichungstag der Anmeldung: 24.01.2024
(73) Patentinhaber: Linde GmbH, 82049 Pullach (DE); Clariant International Ltd., 4132 Muttenz (CH)
(72) Erfinder: ZELLHUBER, Mathieu, 82152 Martinsried (DE); SCHUBERT, Martin, 81375 München (DE); MEISWINKEL, Andreas, 83253 Rimsting (DE); MESTL, Gerhard, 83052 Bruckmühl (DE); WANNINGER, Klaus, 83052 Bruckmühl (DE); SCHECK, Peter, 81925 München (DE); WÖHL, Anina, 81379 München (DE)
(86) Internationale Anmeldenummer: PCT/EP2022/056570
(87) Internationale Veröffentlichungsnummer: WO 2022/194793

(56) Entgegenhaltungen:
- WO-A1-2018/115418
- WO-A1-2019/243480
- DE-A1- 102017 000 861

## Beschreibung

Die vorliegende Erfindung betrifft ein Verfahren zur Herstellung einer Zielverbindung gemäß dem Oberbegriff des Hauptanspruchs.

### Hintergrund der Erfindung

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den jeweiligen Olefinen und zu Wasser umgesetzt. Die vorliegende Erfindung betrifft die oxidative Dehydrierung von Ethan zu Ethylen, nachfolgend auch als ODHE bezeichnet.

Die ODH(E) kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen und der praktisch irreversiblen Wasserbildung keine thermodynamische Gleichgewichtslimitierung. Die ODH(E) kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine in-situ-Regenerierung ermöglicht bzw. bewirkt. Schließlich werden im Gegensatz zum Steamcracken geringere Mengen an wertlosen Nebenprodukten wie Koks gebildet.

Zu weiteren Details bezüglich der ODH(E) sei auf einschlägige Fachliteratur, beispielsweise Ivars, F. und Löpez Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, sowie X. Li, E. Iglesia, Kinetics and Mechanism of Ethane Oxidation to Acetic Acid on Catalysts Based on Mo-V-Nb Oxides, J. Phys. Chem. C, 2008, 112, 15001-15008, verwiesen.

In der WO 2019/243480 A1 wird ein Verfahren zur Herstellung eines oder mehrerer Olefine und einer oder mehrerer Carbonsäuren vorgeschlagen, bei dem ein oder mehrerer Paraffine einer oxidativen Dehydrierung unterworfen wird oder werden. Für die oxidative Dehydrierung wird ein Reaktor mit mehreren Reaktionszonen verwendet, ein Gasgemisch mit dem einen oder den mehreren Paraffinen wird nacheinander durch die Reaktionszonen geführt, und zumindest zwei der mehreren Reaktionszonen werden einer Temperaturbeeinflussung in unterschiedlichem Umfang unterworfen. Insbesondere haben sich für die ODH(E) MoVNb-basierte Katalysatorsysteme als vielversprechend herausgestellt, wie beispielsweise bei F. Cavani et al., "Oxidative dehydrogenation of ethane and propane: How far from commercial implementation?", Catal. Today, 2007, 127, 113-131, erwähnt. Auch zusätzlich Te enthaltende Katalysatorsysteme können verwendet werden. Ist hier von einem "MoVNb-basierten Katalysatorsystem" oder einem "MoVTeNb-basierten Katalysatorsystem" die Rede, sei hierunter ein Katalysatorsystem verstanden, das die genannten Elemente als Mischoxid aufweist, auch ausgedrückt mit MoVNbOₓ bzw. MoVTeNbOₓ. Die Angabe von Te in Klammern steht für dessen optionale Anwesenheit. Die Erfindung kommt insbesondere mit solchen Katalysatorsystemen zum Einsatz.

Bei der ODH werden insbesondere bei Verwendung von MoVNb(Te)Oₓ-basierten Katalysatoren unter industriell relevanten Reaktionsbedingungen signifikante Mengen der jeweiligen Carbonsäuren der eingesetzten Paraffine, im Falle der ODHE insbesondere Essigsäure, als Nebenprodukte gebildet. Für einen wirtschaftlichen Anlagenbetrieb ist daher eine Koppelproduktion von Olefinen und der Carbonsäuren bei Verwendung des beschriebenen Katalysatortyps in der Regel unvermeidlich, eine bevorzugte Bildung von Olefinen allerdings wünschenswert.

Gemäß Stand der Technik sind Umsatz und Selektivität bei der ODH(E) zumeist begrenzt und nur für wenige Katalysatorsysteme, wie insbesondere die genannten MoVNbTeOₓ-Systeme werden in letzter Zeit ausreichend hohe Umsätze und Selektivitäten berichtet, die eine wirtschaftlich sinnvolle technische Umsetzung versprechen, die konkurrenzfähig zu z.B. Steamcracking sein kann. Dennoch ist bislang keine solche technische Umsetzung realisiert und der Stand der Technik beschränkt sich auf Anlagen im Labor- bzw. maximal Pilotmaßstab.

Im Allgemeinen finden bei technisch relevanten oxidativen Prozessen wie z.B. der Herstellung von Maleinsäureanhydrid (MSA) aus Butan, Buten oder Benzol oder auch der zweistufigen Synthese von Acrylsäure aus Propylen über das Intermediat Acrolein sauerstoffarme Bedingungen Verwendung. Es erfolgt üblicherweise Verwendung von sauerstoffabgereicherter Luft als Oxidationsmittel. Beispielhaft sei hier die DE 198 37 519 A1 zur Oxidation von Propan zu Acrolein und/oder Acrylsäure genannt. Eine aktuelle Übersicht zur MSA-Synthese, die ausnahmslos mit Luft als Oxidationsmittel erfolgt, findet sich beispielsweise auch bei P.V. Mangili et al., "Eco-efficiency and techno-economic analysis for maleic anhydride manufacturing processes", Clean Technol. Environ. Policy 2019, 21,1073-1090.

Grundsätzlich wird im Rahmen einer Prozessintensivierung aber auch die Verwendung von Luft oder sauerstoffangereicherter Luft als Oxidationsmittel diskutiert. Nicht zuletzt spielt in diesem Zusammenhang aber immer auch die Vermeidung von explosionsfähigen Gemischen eine Rolle. Auch aus diesem Grund wird vorzugsweise Luft als Oxidationsmittel verwendet.

Die zusätzliche Einspeisung von Stickstoff oder einem anderen Verdünnungsmedium zusätzlich zu Luft führt im Ergebnis ebenfalls zu vergleichbaren Verhältnissen wie mit sauerstoffabgereicherter Luft. Die Einbringung von Stickstoff mit Luft oder sauerstoffan- oder abgereicherter Luft erfordert in der Produktaufreinigung und -abtrennung dann eine Stickstoffabtrennung. Eine solche Stickstoffabtrennung ist zwar insbesondere für schwere Oxygenate bzw. Kohlenwasserstoffe technisch problemlos realisierbar, aufgrund des niedrigen Siedepunktes von Stickstoff oftmals und insbesondere im Fall der ODH(E) aber nur schwierig bzw. aufwendig zu implementieren. Der Aufwand wird dabei entsprechend umso höher, je niedriger siedend das gewünschte Zielprodukt (bei der ODHE eben Ethylen) ist. Im Gegensatz zu MSA und Acrylsäure kann Ethylen nur unter kryogenen Bedingungen auskondensiert werden.

Die EP 2 716 621 A1, die EP 2 716 622 A1, die WO 2018/115416A1, die WO 2018/115418 A1, die WO 2018/082945 A1 und die EP 3 339 277 A1 der Anmelderin offenbaren zwar die Zuführung von reinem Sauerstoff, z.B. gewonnen aus einer destillativen Luftzerlegung, als alternative Möglichkeit neben der Verwendung von Luft bzw. sauerstoffan- oder -abgereicherter Luft, jedoch nicht die damit verbundenen speziellen Anforderungen an die Reaktionsführung und insbesondere die notwendige Abstimmung von Katalysator und Reaktionsführung. Auch die WO 2020/074750 A1 erwähnt die Verwendung von Sauerstoff bzw. sauerstoffangereicherter Luft als Oxidationsmittel, geht aber ebenfalls nicht weiter auf die damit verbundenen Herausforderungen in der technischen Umsetzung ein. Andererseits ist die Bereitstellung von Sauerstoff durch geeignete Verfahren wie destillative Luftzerlegung oder Druckwechseladsorption, wie bereits in den oben zitieren Anmeldungen ausgeführt, eine etablierte Technologie, die nahezu in jedem Maßstab einfach und wirtschaftlich günstig umgesetzt werden kann.

Gemäß Stand der Technik werden stark exotherme Reaktionen wie die ODH(E) vorzugsweise in Festbettreaktoren, insbesondere in gekühlten Rohrbündelreaktoren, durchgeführt. Das Kühlmittel wird dabei im Gleich- oder Gegenstrom zur Flussrichtung des Reaktionseintrittstroms geführt, vorteilhafterweise im Gegenstrom, da hier die abgeführte Wärme aus den späteren Reaktionszonen in den vorderen Reaktionszonen genutzt werden kann. Als Kühlmittel kommen entsprechend dem jeweiligen Temperaturbereich der Reaktion Thermoöle oder insbesondere Salzschmelzen zur Anwendung. Für entsprechende Reaktionen, wozu auch die ODH(E) zählt, ist dabei allgemein die Verwendung eines Reaktorbettes mit mehreren Zonen bekannt. Grundlagen sind z.B. in WO 2019/243480 A1 der Anmelderin beschrieben. Diese Schrift offenbart dabei das Prinzip, dass verschiedene Katalysatorbetten bzw. entsprechende Reaktionszonen, die unterschiedliche Katalysatorbeladungen und/oder Katalysatoraktivitäten pro Raumeinheit aufweisen, zum Einsatz kommen.

Generell erfordern stark exotherme Reaktionen wie vorliegend von Interesse, beispielsweise oxidative Reaktionen wie die ODH, insbesondere die ODHE, aufgrund der Exothermie eine wirkungsvolle Temperaturkontrolle und eine Abführung der entstehenden Reaktionswärme. Diese Temperaturkontrolle ist umso wichtiger, je höher der angestrebte Umsatz ist, da mit zunehmendem Umsatz die freigesetzte Wärmemenge proportional steigt.

Häufig entstehen in entsprechenden Reaktionen neben dem Zielprodukt (im Falle der ODH ein Olefin, bei der ODHE Ethylen) weitere sauerstoffhaltige Spezies, wie z.B. Carbonylverbindungen, Carbonsäuren und/oder Kohlenstoffoxide, d.h. Kohlenmonoxid und/oder Kohlendioxid. Bei zu hohen Temperaturen oder lokalen Temperaturspitzen, die insbesondere durch Temperaturgradienten auftreten bzw. durch diese bewirkt werden können, wird insbesondere die unerwünschte Bildung der Kohlenstoffoxide gefördert. Hier wirken sich also zumeist hohe Temperaturen und/oder lokale Temperaturspitzen, die wie beschreiben mit hohem Umsatz einhergehen, wiederum stark negativ aus.

Eine der wesentlichen Herausforderungen ist daher die Beherrschung der exothermen Reaktion auch bei kommerziell bevorzugten, hohen Umsätzen (z.B. mehr als 40%, 50%, 60%, 70%, 80%, 85% oder 90% pro Durchgang) und hohen Reaktionsgeschwindigkeiten. Für die ODHE in einer erfindungsgemäßen Ausführung werden typischerweise nur um 40%, 50%, 60% oder 66% Umsatz unter Verwendung konzentrierter Feedströme und Verwendung von reinem Sauerstoff erreicht, da der maximale Umsatz durch Wärmeabfuhr und Temperaturerhöhung limitiert wird. Höhere Umsätze führen hier dann zu einem deutlichen Selektivitätsverlust, der i.d.R. keinen wirtschaftlichen Betrieb des Prozesses mehr erlaubt.

Wie beschrieben, erfolgt in anderen Fällen neben einer geeigneten Kühlung des Reaktionsvolumens typischerweise auch eine Verdünnung des Reaktionsgases. Beispielsweise werden höhere Umsätze (von mehr als 70%, 80%, 85% oder 90%) bei der MSA-Synthese nur bei entsprechender Verdünnung und/oder Verwendung von Luft bzw. sauerstoffabgereicherter Luft erreicht. Die genannten Maßnahmen sind in der Praxis, insbesondere der ODH(E), jedoch nur bis zu einem gewissen Grad sinnvoll umsetzbar, da damit sowohl der apparative Aufwand und damit verbunden der Investitionsaufwand für ein solches Verfahren steigt (größere Reaktoren/Behälter/Apparate) und auch der Energieaufwand für Trenn- und Verdichtungsschritte signifikant zunimmt, wodurch nicht nur die Ökobilanz (Kohlendioxid-Fußabdruck) verschlechtert wird, sondern auch die laufenden Kosten eines Verfahrens steigen. Wie beschrieben ist dabei insbesondere die Abtrennung von inerten Verdünnungsmedien (Stickstoff, Argon, Kohlendioxid ...) von niedrig siedenden Komponenten wie Ethylen eine Herausforderung.

Die vorliegende Erfindung stellt sich die Aufgabe, verbesserte und effektivere Möglichkeiten zur Herstellung von Zielverbindungen der genannten Art aufzuzeigen.

### Offenbarung der Erfindung

Die vorstehend genannte Aufgabe wird durch die im unabhängigen Patentanspruch angegeben Maßnahmen gelöst. Bevorzugte Ausgestaltungen sind jeweils Gegenstand der abhängigen Patentansprüche und der nachfolgenden Beschreibung.

Im Gegensatz zu den o.g. genannten etablierten Oxidationsprozessen basiert das erfindungsgemäße Verfahren auf der Verwendung von reinem Sauerstoff als Oxidationsmittel. Dieser reine Sauerstoff lässt sich leicht und preisgünstig aus geeigneten Quellen, wie z.B. destillativen Luftzerlegungsanlagen oder auch Druckwechseladsorption bereitstellen. Ein Aspekt der vorliegenden Erfindung liegt darin, Katalysator und Prozess in optimaler Weise aufeinander abzustimmen und hier besondere Vorteile zu erzielen.

Unter "reinem" Sauerstoff sollen hier auch Gemische mit mindestens 95%, 98%, 99%, 99,5% oder 99,9% Sauerstoffgehalt verstanden werden, insbesondere kann es sich um sogenannten "technischen" Sauerstoff handeln.

Zudem gilt es dem Prozess ein geeignetes Verdünnungsmedium zuzusetzen. Diese Aufgabe nimmt in den zuvor genannten Prozessen mindestens anteilig üblicherweise Stickstoff wahr. Grundsätzlich können auch andere insbesondere gasförmige inerte Verdünnungsmedien zum Einsatz kommen, wobei unter einem "inerten Verdünnungsmedium" hier eine oder ein Gemisch mehrerer Komponenten verstanden werden soll, die in der oxidativen Dehydrierung nicht oder nur in unerheblichem Umfang umgesetzt werden, insbesondere Argon, Helium, Kohlendioxid oder Wasserdampf. Im Rahmen der vorliegenden Erfindung wird hingegen entsprechend der WO 2018/115416 A1, der WO 2018/115418 A1 und der WO 2019/243480 A1 besonders bevorzugt Wasserdampf verwendet, welcher sich durch Kondensation nicht nur besonders leicht und effizient abtrennen lässt, sondern gleichzeitig eine vorteilhafte Selektivitätssteuerung und Moderierung der Katalysatorselektivität ermöglicht. Weiter ermöglicht insbesondere Wasserdampf aufgrund seiner hohen Wärmekapazität eine verbesserte Verteilung der Reaktionswärme über das Reaktorrohr.

Solche für ein kommerzielles Verfahren sehr vorteilhaften Bedingungen wurden bisher nicht für einen industriell relevanten Prozessaufbau für eine oxidative Umsetzung von Kohlenwasserstoffen umgesetzt oder beschrieben.

Die Erfindung schlägt, mit anderen Worten, ein Verfahren zur Herstellung einer Zielverbindung vor, bei dem ein wenigstens eine Eduktverbindung enthaltendes Einsatzgemisch gebildet, auf parallel angeordnete Reaktionsrohre eines oder mehrerer Rohrbündelreaktoren verteilt, und in den Reaktionsrohren einer oxidativen katalytischen Umsetzung unterworfen wird, wobei Ethan als Einsatzverbindung verwendet wird und das oxidative katalytische Verfahren als oxidative Dehydrierung des Ethans durchgeführt wird.

Dem Einsatzgemisch wird erfindungsgemäß Wasserdampf in einer Menge zugegeben, die derart bemessen ist, dass ein Wasserdampfanteil des Einsatzgemischs 5 bis 95 Vol.-% beträgt, dem Einsatzgemisch wird ferner Sauerstoff in Form eines zumindest 95 Vol.-% Sauerstoff enthaltenden Fluids zugegeben, und die oxidative katalytische Umsetzung wird unter Verwendung eines oder mehrerer, die Metalle Molybdän, Vanadium, Niob und optional Tellur enthaltender Katalysatoren durchgeführt, der oder die zumindest teilweise aus den Oxiden der Metalle hergestellt ist oder sind. Wie bereits zuvor ausführlich erläutert, sind derartige Katalysatoren für entsprechende Verfahren besonders vorteilhaft.

Der eine oder die mehreren Katalysatoren ist oder sind erfindungsgemäß in Reaktionszonen des einen oder der mehreren Rohrbündelreaktoren bereitgestellt, die in einer Strömungsrichtung hintereinander angeordnet sind und in der Strömungsrichtung durchströmt werden. Die Reaktionszonen werden jeweils durch entsprechend ausgebildete Abschnitte der Reaktionsrohre gebildet. Der eine oder die mehreren Katalysatoren ist oder sind in den mehreren Reaktionszonen mit einer unterschiedlichen Katalysatorbeladung und/oder einer unterschiedlichen Katalysatoraktivität pro Raumeinheit bereitgestellt, wodurch eine Aktivitätsabstufung zwischen den Reaktionszonen erzielt wird. Optional können die Reaktionszonen auch unterschiedlich beheizt werden.

Eine zumindest eine in der Strömungsrichtung stromabwärts angeordnete Reaktionszone ist dabei insbesondere mit einer höheren Katalysatorbeladung und/oder mit einer höheren Katalysatoraktivität pro Raumeinheit ausgebildet als in einer in der Strömungsrichtung stromaufwärts hiervon angeordneten Reaktionszone.

Besonders vorteilhaft ist die erfindungsgemäß vorgesehene Führung der Reaktion in mehreren Reaktionszonen, die durch Katalysatorlagen unterschiedlicher Aktivitätsabstufungen (insbesondere zunehmende Aktivität in Flussrichtung des Reaktionseinsatzstromes) und/oder unterschiedlich beheizten Zonen ausgebildet werden können (vgl. WO 2019/243480 A1). Dadurch ergibt sich ein "Strecken" des Temperaturhotspots über die Reaktorlänge bzw. ein Verteilen des Temperaturhotspots auf mehrere lokale Hotspots über die Länge des Reaktors, wobei die relative Höhe der jeweiligen Hotspots zur Kühlmitteltemperatur (1. Hotspot) bzw. zur Endtemperatur der vorangegangenen Reaktionszone (Hotspots der folgenden Zonen) immer deutlich kleiner ist als der Temperaturunterschied von der Kühlmitteltemperatur zum globalen Hotspot eines Einlagenkatalysatorbetts bzw. von nur einer Reaktionszone. Dies hat die unten weiter erläuterten Vorteile.

Die Kombination der erfindungsgemäß vorgeschlagenen Prozessparameter - beinhaltend auch ein abgestimmtes Reaktordesign wie nachfolgend weiter ausgeführt sowie die erwähnte Aktivitätsabstufung zwischen den Reaktionszonen - mit einem erfindungsgemäß eingesetzten Katalysator auf der Basis von MoVNbOₓ bzw. insbesondere MoVNbTeOₓ ermöglicht einen hohen Umsatz bei besonders hoher Selektivität und möglichst geringem Inertanteil (hier insbesondere Wasserdampf, Stickstoff bzw. andere insbesondere gasförmige inerte Verdünnungsmedien können komplett vermieden werden) im Feedstrom zu den gewünschten Wertprodukten, insbesondere Ethylen und Essigsäure.

Die erfindungsgemäßen Vorteile werden insbesondere durch eine Kombination dreier Maßnahmen erzielt, die (1) die erwähnten und nachfolgend weiter erläuterte Prozessbedingungen, (2) eine Bereitstellung des Katalysators in mehreren Zonen, und (3) insbesondere eine nachfolgend erwähnte Katalysatorrezeptur umfassen. Eine Steigerung der Selektivität hin zu Ethylen ist, wie auch in den Beispielen erläutert, durch Einhalten einer höheren Mindesttemperatur sowie einer höheren mittleren Temperatur im Katalysatorbett der mehreren Reaktionszonen möglich. Dadurch werden wiederum herausragende Ausbeuten und ein besonders wirtschaftlicher Betrieb des Verfahrens ermöglicht. Der Energieeinsatz und Kohlendioxidemissionen werden minimiert.

Durch den Verzicht auf niedrig siedende Verdünnungsmittel kann eine deutliche Verringerung des Trennaufwandes im Zerlegungs- und Produktaufreinigungsteil erzielt werden. Eine Erhöhung der Betriebssicherheit ergibt sich durch ein geringeres Risiko eines thermischen Durchgehens der Reaktion, wie ebenfalls im Zusammenhang mit den Beispielen erläutert.

Die vorliegende Erfindung führt zu einer stabilen Reaktor- und Katalysatorperformance über einen langen Zeitraum, wie durch entsprechende Versuche belegt.

Die Erfindung kann insbesondere mit spezifischen stündlichen Gas- oder Gewichts-Raumgeschwindigkeiten (GHSV, Gas Hourly Space Velocity; WHSV, Weight Hourly Space Velocity) eingesetzt werden. Die GHSV kann insbesondere bei 400 bis 10 000 (Nm³/h)_{Gas}/m³_{Katalysator} bzw. h⁻¹ und die WHSV insbesondere bei 0,8 bis 25 (kg/h)_{Gas}/kg_{Katalysatoraktivmasse} bzw. h⁻¹ liegen, wobei Nm³ Normkubikmeter bezeichnen. Die GHSV wird insbesondere bei Standardtemperatur (0° C) und -druck (1 bar abs.) bestimmt und bezieht sich, wie der obigen Annahme entnehmbar, auf ein Katalysatorvolumen, wohingegen sich die WHSV auf die Masse des aktiven Katalysators bezieht.

Die vorliegende Erfindung kann die Abtrennung des nicht umgesetzten Teils der Eduktverbindung, insbesondere eines Paraffins wie Ethan bei der ODH(E) in einem Zerlegungsteil und die zumindest teilweise Rückführung in den oder die Reaktoren umfassen.

Prinzipiell erscheint die erfindungsgemäße Ausführung des Verfahrens auch für andere Oxidationsverfahren, insbesondere die Oxidation von Propan/Propylen zu Acrylsäure interessant und bietet Potential.

Vorteilhafterweise beträgt der Wasserdampfanteil des erfindungsgemäß verwendeten Einsatzgemischs 10 bis 50 Vol.-%, insbesondere 14 bis 35 Vol.-%. Es hat sich überraschend gezeigt, dass bei derartigen Wasserdampfanteilen einerseits ein stabiler Katalysatorbetrieb gewährleistet werden kann (eine gewisse untere Grenze für den Wasseranteil im Einsatzstrom ist für einen stabilen Katalysatorbetrieb vorteilhaft, wie bereits in der EP 3 558 910 B1 gezeigt) und andererseits auch die Effizienz des Verfahrens sichergestellt werden kann. Für letzteren Aspekt betrifft dies insbesondere die hier angegebene obere Grenze des Wasserdampfanteils. Wasserdampf dient gleichzeitig Inertmedium bzw. auch als Moderator, jedoch verringert ein zu großer Wasserdampfanteil die Effizienz und somit auch Wirtschaftlichkeit des Verfahrens. Im Rahmen der vorliegenden Erfindung kann Dampf insbesondere als im Wesentlichen einziges Verdünnungsmedium eingesetzt werden. Es wird also insbesondere kein weiteres Inertgas in nennenswerten Anteilen verwendet. Hinsichtlich der Effizienz sind wesentliche Elemente der Energiebedarf zur Dampferzeugung und die Dimensionierung von Apparaten, insbesondere des Dampferzeugers, des Reaktors, nachgelagerter Wärmetauscher und Abscheider. Insbesondere im erfindungsgemäßen Rohrbündelreaktor gilt es eine möglichst hohe Raum-Zeitausbeute an Wertprodukt zu erzielen. Durch eine entsprechende Optimierung des Wasseranteils kann zudem eine möglichst hohe Konzentration an Essigsäure im Kondensat erzielt werden. Somit wird auch hierdurch der apparative und energetische notwendige Aufwand zur weiteren Aufkonzentration von Essigsäure als Wertprodukt minimiert.

Das Einsatzgemisch wird insbesondere derart gebildet, dass ein Verhältnis eines Sauerstoffanteils in dem Einsatzgemisch zu einem Anteil der wenigstens einen Eduktverbindung in dem Einsatzgemisch mindestens 0,20, 0,25, 0,30 oder 0,35 und bis zu 0,5 oder 1,0 beträgt. Bei derartigen Sauerstoffanteilen kann insbesondere bei einem angestrebten Ethanumsatz im Bereich von 40 bis 60% und ohne Zusatz weiteren Verdünnungsmediums als dem zuvor genannten Wasserdampf eine sehr hohe Gesamtselektivität zu den bevorzugten Wertprodukten Ethylen und Essigsäure erzielt werden kann. Es werden also insbesondere die Anteile an den ungewünschten Nebenprodukten Kohlenmonoxid und Kohlendioxid auf einen Anteil begrenzt, der mit technisch üblichen Mitteln beherrschbar ist. Dies betrifft einerseits zunächst die Wärmeabfuhr im Reaktor aufgrund der starken Exothermie bei der Bildung von Kohlenmonoxid und Kohlendioxid. Wie bereits erwähnt, wird der Sauerstoff in einer Reinheit von zumindest 95 Vol.-% zugeführt, so dass der zuvor genannte Wasserdampfanteil im Einsatzgemisch der wesentliche Inertanteil im Einsatzgemisch ist. Die Verdünnung der wesentlichen Reaktivkomponenten Ethan und Sauerstoff ist also im Vergleich zu anderen literaturbekannten Systemen minimiert. Eine optimale Abstimmung der Katalysatoreigenschaften und der Prozessbedingungen ist somit also wesentlich für die praktische Durchführung der oxidativen Dehydrierung unter technischen Bedingungen, um die angestrebten Anforderungen hinsichtlich hoher Wertproduktausbeute und dafür notwendiger Reaktionskontrolle und Wärmeabfuhr zu gewährleisten. Ebenso reduziert sich aber auch der notwendige Aufwand zur Entfernung von Kohlenmonoxid und Kohlendioxid im nachgeschalteten Zerlegungsteil und die gebildeten Anteile von Kohlenmonoxid und Kohlendioxid können mit technisch üblichen Verfahrensschritten entfernt werden. Hierbei handelt es sich beispielsweise um eine Demethanisierung, in der das Kohlenmonoxid mit entfernt wird, oder eine katalytische Umsetzung des Kohlenmonoxids und um eine Amin- oder Laugewäsche für die Entfernung von Kohlendioxid.

Das Einsatzgemisch wird im Rahmen des erfindungsgemäßen Verfahrens insbesondere derart gebildet, dass ein Verhältnis des Wasserdampfanteils in dem Einsatzgemisch zu einem Anteil der wenigstens einen Eduktverbindung in dem Einsatzgemisch mindestens 0,23 beträgt.

Wie erwähnt, eignet sich die Erfindung insbesondere für die Verwendung im Zusammenhang mit der ODH, insbesondere der ODHE, so dass insbesondere Ethan als Einsatzverbindung verwendet wird und das oxidative katalytische Verfahren als oxidative Dehydrierung des Ethans durchgeführt wird.

Die vorliegende Erfindung sieht insbesondere vor, das oxidative katalytische Verfahren bei einer Temperatur des oder der Katalysatoren in einem Bereich zwischen 240 und 500 °C, insbesondere zwischen 280 und 450 °C, weiter insbesondere zwischen 300 und 400 °C durchzuführen, und/oder dieses mit einem Gesamtdruck des Einsatzgemischs an einem Eintritt des oder der Rohrbündelreaktoren von 1 bis 10 bar (abs.), insbesondere 2 bis 6 bar (abs.) durchzuführen.

In dem erfindungsgemäßen Verfahren werden die Reaktionsrohre insbesondere unter Verwendung eines oder mehrerer, die Reaktionsrohre umfließender Kühlmedien gekühlt, wie bereits zuvor erwähnt.

In Ausgestaltungen der Erfindung können unterschiedliche Abschnitte der Reaktionsrohre unter Verwendung unterschiedlicher Kühlmedien, unter Verwendung desselben Kühlmediums in unterschiedlichen Kühlmedienkreisläufen, und/oder unter Verwendung desselben oder unterschiedlicher Kühlmedien in unterschiedlichen oder gleichen Strömungsrichtungen gekühlt werden.

Im Rahmen der Erfindung ist bzw. sind der oder die Katalysatoren in unterschiedlichen Zonen der Reaktionsrohre mit unterschiedlichen Aktivitäten bereitgestellt, wie zuvor erläutert. Insbesondere wird dabei ein maximaler Temperaturunterschied von 60 K, 55 K, 50 K, 45 K oder 40 K zwischen einem oder mehreren Temperaturhotspots, insbesondere aller Temperaturhotspots der erwähnten Reaktionszonen, und einer Kühlmitteltemperatur, beispielsweise einer Salztemperatur, eingehalten. Dies führt zur Vermeidung sehr hoher lokaler Temperaturen. Das Einhalten dieser maximalen Temperaturunterschiede wird durch die erwähnte Bereitstellung der Katalysatoren mit unterschiedlichen Aktivitäten erzielt, die Aktivitäten werden also derart mit ihren unterschiedlichen Aktivitäten bereitgestellt, dass ein oder mehrere der Temperaturhotspots, insbesondere alle, den erwähnten maximalen Temperaturunterschied aufweisen. Ein "Temperaturhotspot" ist die Position in der jeweiligen Zone, die die höchste Temperatur aufweist.

Das katalytisch aktive Material des oder der Katalysatoren ist damit aus kommerziell in großen Mengen und zu günstigen Preisen erhältlichen Vorstufen herstellbar. Die Nachteile der Herstellung aus (wasser-) löslichen Vorstufen der Metalle, wie z.B. Ammoniumheptamolybdat oder Vanadylsulfat, können auf diese Weise vermieden werden. Anstelle von Tellursäure kann Telluroxid verwendet werden. Insbesondere kann das katalytisch aktive Material (vollständig) unter Verwendung der auch nachfolgend noch erwähnten Oxide hergestellt werden.

Der oder die Katalysatoren wird bzw. werden insbesondere unter Verwendung einer Hydrothermalsynthese, insbesondere in einem Autoklaven und insbesondere unter Verwendung eines Drucks im Bereich von 5 bis 50 bar (abs.), insbesondere von 10 bis 35 bar (abs.), weiter insbesondere von 15 bis 30 bar (abs.), und einer Temperatur im Bereich von 150 bis 280 °C, insbesondere von 150 bis 230 °C, weiter insbesondere von 170 bis 210°C hergestellt.

Weiter wird bzw. werden der oder die Katalysatoren insbesondere durch Kristallisation unter hydrothermalen Bedingungen unter Verwendung von Molybdäntrioxid, Divandadiumpentoxid, Diniobpentoxid und optional Tellurdioxid und unter Verwendung von Oxo-Liganden hergestellt, wobei die Oxo-Liganden insbesondere jeweils mindestens zwei Sauerstoffatome aufweisen und aus Carbonsäuren und Alkoholen, insbesondere aus Oxalsäure, Zitronensäure und 1,2-Alkandiolen ausgewählt sind.

Der oder die Katalysatoren wird bzw. werden im Rahmen der vorliegenden Erfindung insbesondere durch Verpressen in eine Partikelform gebracht.

Ein katalytisch aktives Material, der eigentliche Katalysator, kann dabei insbesondere zusammen mit einem katalytisch inaktiven Material, das selbst nicht katalytisch wirkt, aber zusammen mit dem Katalysator bereitgestellt ist, verwendet werden. Das katalytisch inaktive Material kann beispielsweise Silika (SiO₂), Aluminiumoxid (Al₂O₃), Siliziumcarbid (SiC) oder Graphit sein. Insbesondere Siliziumcarbid und Graphit sind für (stark) exotherme Reaktionen wie der Oxidation von Alkanen, insbesondere der ODH-E, sehr vorteilhafte inerte Materialien, da diese neben dem Effekt der Verdünnung besonders gut wärmeleitend sind und so auch zu einem effektiven Wärmemanagement der Reaktion beitragen. Für eine Formgebung (Tablettierung) der Katalysatoren wird zusätzlich Wachs benötigt, das aber nach erfolgter Formgebung ausgebrannt wird und somit im eigentlichen Katalysator nicht mehr vorhanden ist aber stattdessen entsprechende Poren hinterlässt, die für die Zugänglichkeit der Reaktanden zu den katalytisch aktiven Zentren wichtig sind. Oben erwähnte Inertmaterialien können für eine Tablettierung oder als Gerüstmaterialien für geeignete Katalysatorformkörper beliebiger Art verwendet werden oder es kann sich um weitere, nicht mit katalytisch aktivem Material ausgestattete Körper handeln.

Der oder die Katalysatoren weist bzw. weisen insbesondere eine katalytisch inaktive Komponente, insbesondere ein katalytisch inaktives Metalloxid, auf, und in den Reaktionsrohren sind vorteilhafterweise Katalysezonen bereitgestellt, in denen der oder die Katalysatoren in unterschiedlichen Mengen mit dem inaktiven Metalloxid verdünnt ist bzw. sind.

Eine Anlage zur Herstellung einer Zielverbindung mit einem oder mehreren, parallel angeordnete Reaktionsrohre aufweisenden Rohrbündelreaktoren, die dafür eingerichtet ist, ein wenigstens eine Eduktverbindung enthaltendes Einsatzgemisch zu bilden, auf Reaktionsrohre des oder der Rohrbündelreaktoren zu verteilen, und in den Reaktionsrohren einer oxidativen katalytischen Umsetzung zu unterwerfen, ist ebenfalls hierin beschrieben.

Diese ist dafür eingerichtet dem Einsatzgemisch Wasserdampf in einer Menge zuzugeben, die derart bemessen ist, dass der Wasserdampfanteil des Einsatzgemischs 5 bis 95 Vol.-% beträgt, und dem Einsatzgemisch Sauerstoff in Form eines zumindest 95 Vol.-% Sauerstoff enthaltenden Fluids zuzugeben, wobei für die oxidative katalytische Umsetzung in den Reaktionsrohren ein oder mehrerer, die Metalle Molybdän, Vanadium, Niob und optional Tellur enthaltende Katalysatoren verwendet werden.

Im Gegensatz zu den o.g. genannten etablierten Oxidationsprozessen basiert das erfindungsgemäße Verfahren auf der Verwendung von reinem Sauerstoff als Oxidationsmittel. Dieser reine Sauerstoff lässt sich leicht und preisgünstig aus geeigneten Quellen, wie z.B. destillativen Luftzerlegungsanlagen oder auch Druckwechseladsorption bereitstellen.

Die vorliegende Erfindung löst die Aufgabe, einen für die verwendeten Bedingungen angepassten und optimierten Katalysator zu finden. Hier kommen wie beschrieben grundsätzlich zunächst MoVNbOₓ- und insbesondere MoVNbTeOₓ-Katalysatoren in Betracht. Hier gilt es gemäß Stand der Technik, nicht nur einen möglichst hohen Anteil an so genannter M1-Phase bereitzustellen, sondern ebenfalls die Aktivität und Selektivität genau auf die gewünschten erfindungsgemäßen Prozessbedingungen abzustimmen.

Vorteile und Merkmale der Erfindung und besonders bevorzugter Ausgestaltungen der Erfindung werden nachfolgend nochmals zusammengefasst.

Üblicherweise werden diese Katalysatoren hergestellt, indem Lösungen der löslichen Metallsalze, wie Ammoniumheptamolybdat, Vanadylsulfat, Tellursäure und Ammonium-Nioboxalat vereinigt werden. Die vereinigte Lösung kann sprühgetrocknet werden. Oft wird auch zur Erhöhung der katalytisch aktiven Phase (M1) eine weitere Kristallisationszeit unter hydrothermalen Bedingungen (über 100 °C in Wasser im Autoklaven) angeschlossen. Danach wird getrocknet. Der oxidische Katalysator bildet sich dann aber bei einer Kalzinierung unter Inertgas bei über 550 °C. Diese Verfahren sind in der Fachliteratur beschrieben, siehe u.a. A. Celaya Sanfiz et al., "Preparation of Phase-Pure M1 MoVTeNb Oxide Catalysts by Hydrothermal Synthesis-Influence of Reaction Parameters on Structure and Morphology", Top. Catal. 50, 2008, 19-32 oder D. Melzer et al., "Atomic-Scale Determination of Active Facets on the MoVTeNb Oxide M1 Phase and Their Intrinsic Catalytic Activity for Ethane Oxidative Dehydrogenation", Angew. Chem. Int. Ed. 55, 2016, 8873-8877. Ein solcher Katalysator kann ebenfalls Bestandteil der Erfindung sein.

Der erfindungsgemäß eingesetzte oxidative Prozess, nämlich ein ODH(E)-Prozess, kann überraschenderweise in besonders vorteilhafter Weise mit einem im Vergleich zu einem typischerweise verwendeten Katalysator weniger aktiven Katalysator realisiert werden. In der Regel ist ein solch weniger aktiver Katalysator aber wirtschaftlich unvorteilhaft, da niedrigere Ausbeuten erzielt werden oder z.B. höhere Reaktionstemperaturen eingestellt werden müssen, die zwar die Aktivität steigern, sich jedoch wiederum negativ auf die Selektivität auswirken.

Des Weiteren wurde überraschenderweise gefunden, dass ein besonders geeigneter, d.h. weniger aktiver Katalysator, hergestellt werden kann, wenn man in der Katalysatorsynthese von den entsprechenden Metalloxiden ausgeht und nicht wie üblich von löslichen Komponenten und/oder ggf. von Tellurdioxid. Ein solcher Katalysator kann insbesondere durch Kristallisation unter hydrothermalen Bedingungen aus den Oxiden der Metalle unter Verwendung von Oxo-Liganden hergestellt werden, wie unten noch genauer spezifiziert.

Überraschend ist, dass ein derartig hergestellter Katalysator deutlich selektiver ist als bei Verwendung anderer Katalysatoren der gleichen Zusammensetzung. Die Oxo-Liganden haben alle mindestens zwei Sauerstoffatome, welche koordinieren können und die Oxo-Liganden sind aus der Gruppe der Carbonsäuren und Alkohole ausgewählt. Insbesondere kann es sich bei dem Oxo-Liganden um Oxalsäure, Zitronensäure und ein 1,2-Alkandiol wie (Ethylen-)Glykol handeln. Die Hydrothermalsynthese findet vorteilhafterweise in einem geschlossenen Autoklaven statt im Bereich von 10 bis 30 bar (abs.) und im Bereich von 150 bis 230 °C (besonders bevorzugt von 170 bis 210 °C). Ein derartig hergestellter Katalysator ist in der DE 10 2017 000 861 A1 beschrieben. Dort wurde aber gefunden, dass der Katalysator unter anderen, wasserfreien, sehr verdünnten Prozessbedingungen eine etwas höhere Aktivität als vergleichbare Katalysatoren aus löslichen Vorstufen aufweist. Eine höhere Selektivität wird dort nicht berichtet. Daher ist es umso überraschender, dass dieser Katalysator unter den neuen erfindungsgemäßen Prozessbedingungen ohne Inertgas und mit Wasserdampf im Eduktgas eine geringere Aktivität, aber eine höhere Selektivität aufweist.

Im Ergebnis führt die Verwendung eines solchen Katalysators unter den erfindungsgemäßen Prozessbedingungen, nämlich in der ODH(E), gerade zu erhöhter Selektivität zum Zielprodukt, d.h. bei der ODH(E) zu Ethylen, da die Aktivität zwar geringer ist und damit eine höhere Temperatur notwendig ist, gleichzeitig aber eine besonders hohe Selektivität erreicht wird. Grundlagen zum Hintergrund sind beispielsweise in der WO 2018/115416 A1 beschrieben, wo der Effekt genutzt wird, um das Produktverhältnis von Ethylen und Essigsäure innerhalb gewisser Grenzen durch Anpassung der Feedgaszusammensetzung und insbesondere des Wasseranteils bedarfsgerecht anzupassen. In der vorliegenden Erfindung wird der Effekt jedoch insgesamt genutzt, um gezielt den Anteil von Wertprodukt (bei der ODHE insbesondere Ethylen) zu steigern.

Die erfindungsgemäß vorgesehene Führung der Reaktion in mehreren Reaktionszonen, durch Katalysatorlagen unterschiedlicher Aktivitätsabstufungen, hat insbesondere die folgenden Vorteile:
- Erhöhung der Betriebssicherheit durch geringeres Risiko eines thermischen Durchgehens der Reaktion
- Steigerung der Selektivität hin zu Ethylen durch Einhalten einer höheren Mindesttemperatur sowie einer höheren mittleren Temperatur im Katalysatorbett der mehreren Reaktionszonen
- Steigerung des Umsatzes pro Durchgang und verbunden mit der erhöhten Selektivität zu Ethylen
- Steigerung der Ethylenausbeute durch ermöglichten Betrieb bei höheren Reaktor-/Kühlmitteltemperaturen aufgrund der erhöhten Betriebssicherheit/geringerem Risiko eines thermischen Durchgehens

Im Rahmen der Erfindung wird also offenbart, dass sich ein Verfahren für eine selektive Oxidation von Kohlenwasserstoffen besonders vorteilhaft ausführen lässt, indem Aspekte von Reaktordesign, Reaktionsführung und Katalysatorpräparation optimal miteinander verknüpft werden. Bei einer solchen selektiven Oxidation von Kohlenwasserstoffen handelt es sich erfindungsgemäß um eine oxidative Dehydrierung von Ethan.

### Ausführungsbeispiele

Die Erfindung wird nachfolgend anhand von Beispielen, die Ausgestaltungen der Erfindung entsprechen, und nicht erfindungsgemäßen Vergleichsbeispielen sowie zugehörigen Figuren und Tabellen weiter erläutert.
Figur 1A veranschaulicht unterschiedliche Katalysatoraktivitäten bei unterschiedlich hergestellten Katalysatoren zum teilweisen Einsatz in der Erfindung.
Figur 1B veranschaulicht Temperaturprofile für unterschiedlich hergestellte Katalysatoren zum teilweisen Einsatz in der Erfindung.
Figur 2 veranschaulicht Langzeitstabilitätsdaten.
Figur 3 veranschaulicht eine Anlage gemäß einer Ausgestaltung der vorliegenden Erfindung in vereinfachter schematischer Darstellung.
Figur 4 veranschaulicht einen Reaktor gemäß einer Ausgestaltung der vorliegenden Erfindung in vereinfachter schematischer Darstellung.

### Vergleichsbeispiel 1

Als Beispiel für die üblicherweise angewandte Synthese von MoVNbOₓ- und MoVNbTeOₓ-Katalysatoren wurde ein nachfolgend mit "Katalysator 1" bezeichneter Katalysator ausgehend von löslichen Ausgangsverbindungen hergestellt, wie bei Melzer et al. (s.o., "Supplementary Material") beschrieben.

### Vergleichsbeispiel 2

Als Beispiel für einen von Tellurdioxid ausgehenden Katalysator wurde ein nachfolgend als "Katalysator 2" hergestellter Katalysator auf Grundlage der DE 10 2017 000 848 A1 hergestellt, wie nachfolgend beschrieben.

In einem Autoklaven (40 L) wurden 3,3 L destilliertes Wasser vorgelegt und unter Rühren auf 80°C erhitzt. Währenddessen wurden 725,58 g Ammoniumheptamolybdat-Tetrahydrat hineingegeben und gelöst ("AHM-Lösung"). In zwei 5-L-Bechergläsern wurden jeweils 1,65 L destilliertes Wasser unter Rühren auf einem Magnetrührer mit Temperaturregelung ebenfalls auf 80°C erhitzt. In diese Bechergläser wurden dann jeweils 405,10 g Vanadylsulfathydrat (Vanadiumgehalt 21,2%) und 185,59 g Ammoniumnioboxalat (Niobgehalt 20,6%) zugegeben und gelöst ("V-Lösung" und "Nb-Lösung").

65,59 g Tellurdioxid wurden am Vortag 3 h in 200 g destilliertem Wasser unter Verwendung einer Kugelmühle gemahlen und mit 1,45 L destilliertem Wasser in ein Becherglas überführt ("Te-Suspension").

Nun wurden nacheinander die V-Lösung in die AHM-Lösung hineingepumpt, dann die am Vortag gemahlene Te-Suspension zugegeben, 1 h bei 80 °C weitergerührt und zum Schluss die Nb-Lösung in die AHM-Lösung mittels einer Schlauchpumpe gepumpt. Die entstandene Suspension wurde nun 10 min bei 80 °C weitergerührt, wobei die Geschwindigkeit des Rührers bei der Fällung 90 rpm betrug.

Anschließend wurde mit Stickstoff überlagert, indem im Autoklaven mit Stickstoff ein Druck bis ca. 6 bar aufgebaut und das Ablassventil so weit geöffnet wurde, dass der Autoklav unter Druck mit Stickstoff durchströmt wurde (5 min). Am Ende wurde der Druck, über das Entlüftungsventil, bis auf 1 bar Restdruck wieder abgelassen.

Die Hydrothermalsynthese im 40-L-Autoklaven wurde bei 175 °C für 20 h (Aufheizzeit: 3 h) mit einem Ankerrührer bei einer Rührergeschwindigkeit von 90 rpm durchgeführt. Nach der Synthese wurde mit Hilfe einer Vakuumpumpe mit Blaubandfilter abfiltriert und der Filterkuchen mit 5 Liter destilliertem Wasser gewaschen.

Die Trocknung erfolgte bei 80°C im Trockenschrank für 3 Tage und anschließend wurde in einer Schlagmühle gemahlen, wobei eine Feststoff-Ausbeute von 0,8 kg erreicht wurde. Die Kalzinierung erfolgte bei 280 °C für 4 h im Luftstrom (Heizrate 5 °C/min, 1 L/min Luft). Die Aktivierung erfolgte in der Retorte bei 650 °C für 2 h im Stickstoffstrom (Heizrate 5 °C/min, 0,5 L/min Stickstoff).

### Vergleichsbeispiel 3

Als Beispiel für einen von den Metalloxiden ausgehenden Katalysator wurde ein nachfolgend als "Katalysator 3" bezeichneter Katalysator auf Grundlage der DE 10 2017 000 861 A1 hergestellt, wie nachfolgend beschrieben.

Tellurdioxid wurde in 200 g destilliertem Wasser aufgeschlämmt und in einer Planetenkugelmühle mit 1-cm-Kugeln (Zirkondioxid) gemahlen. Anschließend wurde die Portion mit 500 ml destilliertem Wasser in ein Becherglas überführt. Diniobpentoxid wurde in 200 g destilliertem Wasser aufgeschlämmt und in der gleichen Kugelmühle gemahlen. Anschließend wurde die Portion mit 500 ml destilliertem Wasser in ein Becherglas überführt. Am nächsten Morgen wurde auf 80 °C aufgeheizt, 107,8 g Oxalsäure-Dihydrat in die Diniobpentoxid-Suspension gegeben und für ca. 1 h gerührt. In einem Autoklaven (40 Liter) wurden 6 L destilliertes Wasser vorgelegt und unter Rühren (Geschwindigkeit des Rührers 90 rpm) auf 80°C erhitzt.

Hatte das Wasser die Temperatur erreicht, wurden nacheinander 61,58 g Zitronensäure, 19,9 g Ethylenglycol, 615,5 g Molybdäntrioxid, 124,5 g Divanadiumpentoxid, das gemahlene Tellurdioxid und das gemahlene Diniobpentoxid in Oxalsäure zugegeben. 850 ml destilliertes Wasser wurden zum Überführen und Spülen der Gefäße verwendet. Die komplette Wassermenge im Autoklav beträgt 8,25 Liter. Anschließend wurde mit Stickstoff überlagert. Es wurde eine Hydrothermalsynthese im 40-L-Autoklaven bei 190 °C/48 Std durchgeführt. Nach der Synthese wurde mit Hilfe einer Vakuumpumpe mit Blaubandfilter abfiltriert und der Filterkuchen mit 5 L destilliertem Wasser gewaschen.

Die Trocknung erfolgte bei 80 °C im Trockenschrank für 3 Tage und anschließend wurde das Produkt in einer Schlagmühle gemahlen. Es wurde eine Feststoff-Ausbeute von 0,8 kg erreicht.

Die anschließende Kalzinierung erfolgte bei 280 °C für 4h an Luft (Heizrate 5 °C/min, 1 L/min Luft). Die Aktivierung erfolgte in einer Retorte bei 600°C für 2h (Heizrate 5 °C/min, 0,5 L/min Stickstoff).

### Vergleichsbeispiel 4

Die wie zuvor beschrieben hergestellten Katalysatorpulver wurden mit 2% Graphit Timerex T44, 10% Siliziumdioxid Siloid C809 Pulver und mit 10% Wachs gemischt, kompaktiert und dann in 3×3mm-Tabletten tablettiert. Diese Tabletten wurden dann gespalten und eine Fraktion von 1 bis 2 mm als Granulat für den Test verwendet. Anschließend wurde noch das Wachs bei 350°C unter Luft ausgebrannt.

### Vergleichsbeispiel 5

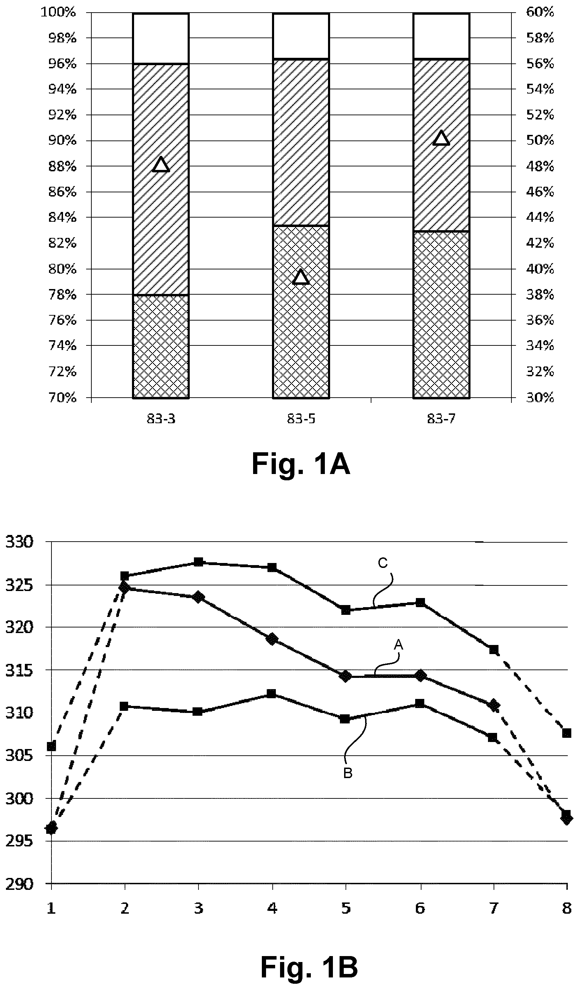

Die so hergestellten Katalysatoren wurden in einer Versuchsanlage hinsichtlich ihrer Aktivität bzw. ihres Umsatz-Selektivitätsverhaltens untersucht. Der Reaktor (nutzbare Länge 0,9 m, Innendurchmesser des Reaktionsraumes 10 mm) ist als Doppelrohr ausgeführt. Die Beheizung bzw. Kühlung erfolgt mit Hilfe eines Thermoölbades, wobei das Thermoöl durch den Außenraum des Reaktors gepumpt wird und somit den Innenraum/Reaktionszone beheizt bzw. auch gleichzeitig kühlt (die Umsetzung ist eine exotherme Reaktion). Die exakten Testbedingungen sind in Tabelle 1 aufgeführt. Ergebnisse sind in Tabelle 2 und Figur 1 dargestellt.

Die Figur 1A veranschaulicht dabei die Selektivitäten (linke Vertikalachse; Kreuzschraffur: Ethylen, Diagonalschraffur: Essigsäure, ohne Füllung: Kohlenstoffoxide) und Umsetzungen (rechte Vertikalachse; Dreiecke) der Katalysatoren gemäß den in Tabelle 1 dargestellten Versuchspunkten A, B und C dargestellt sind.

An den Ergebnissen ist folgendes ersichtlich:
- Der Katalysator 3 auf Basis der Metalloxide zeigt eine um etwa 19 % geringere Aktivität als der Katalysator 2 auf Basis der löslichen Präkursoren und Tellurdioxid (siehe Tabelle 2), d.h. geringerer Ethanumsatz bei gleicher Katalysatorbetteintrittstemperatur bzw. gleicher Kühlmitteltemperatur
- Bei gleichem Umsatz weist der Katalysator 3 basierend auf den reinen Oxiden gegenüber dem Katalysator 2 bei gleicher Gesamtselektivität von mehr als 96% zu den kommerziellen Wertprodukten Ethylen und Essigsäure eine um ca. 5 %-Punkte höhere Selektivität zu Ethylen (und entsprechend 5 %-Punkte niedrigere Selektivität zu Essigsäure) auf, nämlich ca. 83 % vs. ca.78% Selektivität zu Ethylen (Katalysator 3 vs. Katalysator 2) und ca. 13 % vs. ca. 18 % Selektivität zu Essigsäure (Katalysator 3 vs. Katalysator 2), vgl. Figur 1 links
- Flacheres Temperaturprofil (selbst bei höherer Katalysatorbetteintrittstemperatur) von Katalysator 3 vs. Katalysator 2 (Figur 1 rechts) aufgrund der geringeren Aktivität und geringeren Selektivität zu Essigsäure (Die Ethanoxidation hin zu Essigsäure ist deutlich exothermer als die Oxidation von Ethan zu Ethylen; Standardreaktionsenthalpie Ethan zu Ethylen -105 kJ/mol, Standardreaktionsenthalpie Ethan zu Essigsäure -590 kJ/mol
- Verringerung der Gefahr des thermischen Durchgehens des Katalysatorbetts oder eines Teils des Katalysatorbetts bzw. einer Reaktionszone in einem kommerziellen Reaktor
- Ein Katalysator 3 hergestellt aus den Oxiden zeigt unter diesen Bedingungen also insbesondere mit viel Wasserdampf) eine geringere Aktivität. Zur Erzielung des gleichen Umsatzes muss aufgrund dieser geringeren Aktivität also eine höhere Katalysatorbetteintrittstemperatur eingestellt werden, die zugleich auch eine höhere mittlere bzw. Mindest-Katalysatorbetttemperatur bedingt. Überraschenderweise zeigt sich nun aber, dass mit Katalysator 3 bei gleichem Umsatz eine höhere Selektivität erreicht wird.
- Überraschenderweise stellt sich also der vermeintliche Nachteil einer geringeren Aktivität eines über die reinen Oxide hergestellten Katalysators im Sinne der Erfindung als besonders vorteilhaft heraus, da die verringerte Aktivität dazu führt, dass der Prozess bei etwas erhöhten Temperaturen betrieben werden kann/muss, was im Ergebnis dann zu einer erhöhten Ausbeute an besonders bevorzugten Wertprodukt Ethylen führt.

**Tabelle 1**

| **Bedingungen/Versuchspunkt** | **A** | **B** | **C** |
|---|---|---|---|
| Katalysator | **Katalysator 2** | **Katalysator 3** | |
| Katalysatormasse [g] | 48,02 | | |
| Anteil Binder [Gew.-%] | 10 | | |
| Katalysatorform | Geviertelte 3x3 mm Tabletten | | |
| Systemdruck [bara] | 3,5 | | |
| WHSV [g_{C2H6}/(g_{cat}*h)] | 0,8 | | |
| Öltemperatur [°C] | 298 | 298 | 307,7 |
| Mittlere Katalysatorbetttemperatur [°C] | 317,7 | 310,0 | 323,7 |
| O₂/C₂H₆ [mol/mol] | 0,373 | 0,373 | |
| H₂O/C₂H₆ [mol/mol] | 0,234 | 0,286 | |
| Einsatzzusammensetzung [mol%] | | | |
| C₂H₆ | 61,4 | 60,6 | |
| O₂ | 24,2 | 22,0 | |
| H₂O | 14,4 | 17,4 | |

**Tabelle 2**

| **Bedingungen / Versuchspunkt** | **Kat. 2** | **Kat. 3** |
|---|---|---|
| | (Vers.-Pkt. A) | (Vers.-Pkt. B) |
| Katalysatoraktivität [g_{C2H6-Umsatz.}/(g_{Kat}×h)] | 0,378 | 0,305 |
| Relative Katalysatoraktivität [%] | 100 | 81 |

Es ergibt sich ein flacheres Temperaturprofil (selbst bei höherer Katalysatorbetteintrittstemperatur) von Katalysator 3 vs. Katalysator 2 aufgrund der geringeren Aktivität und geringeren Selektivität zu Essigsäure. Durch diesen Effekt kann eine Verringerung der Gefahr des thermischen Durchgehens des Katalysatorbetts oder eines Teils des Katalysatorbetts bzw. einer Reaktionszone in einem kommerziellen Reaktor erreicht werden.

Dieser Umstand ist in Figur 1B veranschaulicht, in der für Messpunkte vor (Messpunkt 1) und nach (Messpunkt 8) einem Katalysatorbett von ca. 60 cm sowie Messpunkte (Messpunkte 2 bis 7) innerhalb des Katalysatorbetts auf der Horizontalachse die entsprechenden Temperaturen in °C auf der Vertikalachse aufgetragen sind.

### Beispiel 1

Es wurde ein Langzeittest eines erfindungsgemäß eingesetzten Katalysators in einem Pilotreaktor durchgeführt. Mit der oben beschriebenen optimierten Katalysatorrezeptur entsprechend Katalysator 3 wurde eine entsprechende Menge Katalysator hergestellt, um einen Reaktor im Pilotmaßstab zu füllen.

Der verwendete Pilotreaktor ist ein mit einer Salzschmelze gekühlter Festbettreaktor. Es handelt sich dabei um den gleichen Pilotreaktor, mit dem die in der WO 2019/243480 A1 beschriebenen Ergebnisse erzielt wurden. Der Pilotreaktor ist als ein Rohr-in-Rohr Reaktor ausgeführt, wobei das Innenrohr mit dem KatalysatorFestbett befüllt ist (Reaktionsraum). Zwischen der Wandung des Reaktionsraumes und dem Außenrohr befindet sich der Kühlmittelraum, d.h. dieser Raum wird mit dem Kühlmittel, hier mit einer flüssigen Salzschmelze, im Gegenstrom zur Flussrichtung des Reaktionseinsatzstromes durchströmt. Bei der Salzschmelze handelt es sich um eine Mischung aus Natriumnitrit, Natriumnitrat sowie Kaliumnitrat. Die Dimensionen (d.h. Länge, Innendurchmesser und Wandstärke) des Reaktionsraumes des Pilotreaktors entsprechen den typischen Dimensionen eines einzelnen Rohres aus einem typischen kommerziellen (großtechnischen) Rohrbündelreaktors. Somit ist der Pilotreaktor als ein echtes Abbild einer großtechnischen Anlage anzusehen (also Scale-up weg vom Labormaßstab), da sich in diesem Pilotreaktor aufgrund seiner Geometrie die gleichen Verhältnisse (Strömungsfeld, Temperatur-bzw. Temperaturgradienten, Druckgradienten etc.) wie in einem technischen Rohrbündelreaktor einstellen und sich somit die Reaktion unter realen technischen Bedingungen testen lässt.

Für den Testbetrieb wurde der Pilotreaktor mit einem hinsichtlich der katalytischen Aktivität dreistufigen Katalysatorbett gefüllt. Dabei war das katalytisch aktive Grundmaterial für jede Stufe exakt das gleiche. Das Bett war dabei so angeordnet, dass die katalytische Aktivität in Flussrichtung des Reaktionseinsatzstromes zunahm. Die unterschiedliche Aktivitätsabstufung wurde (wie auch in der WO 2019/243480 A1 beschrieben) durch Katalysatorformkörper (Ringe) mit unterschiedlich hoher Beimengung an Binder, der zur Formung der Formkörper benötigt wird, zum exakt gleichen katalytisch aktiven Grundmaterial erreicht. Der Binder fungiert also gleichzeitig auch als Verdünnungsmittel des aktiven Katalysatormaterials. Jede Katalysatorschicht wies die gleiche Höhe und somit das gleiche Volumen auf. Stromauf und stromab des dreistufigen Katalysatorbetts befand sich jeweils eine Schüttung aus Inertmaterial gleicher Form und Größe wie die Katalysatorformkörper.

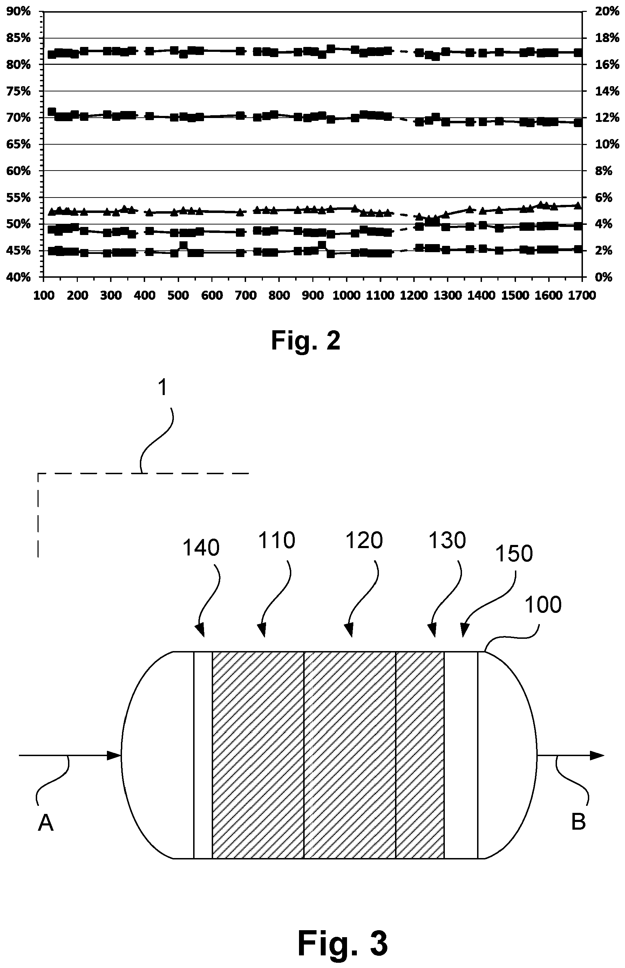

Der Pilotreaktor wurde sodann über einen Zeitraum von ca. 1700 h (ca. 71 Tage) mit einem Reaktionseinsatzstrom im Wesentlichen bestehend aus Ethan, Sauerstoff sowie Wasser(dampf) betrieben. Die genauen Reaktionsbedingungen sind in Tabelle 3 aufgeführt. Über den gesamten Testzeitraum war eine gleichbleibende, stabile und sehr gute Reaktor- bzw. Katalysatorperformance hinsichtlich Ethanumsatz und Selektivitäten zu den gewünschten kommerziellen Wertprodukten Ethylen und Essigsäure zu beobachten wie auch aus Figur 2 ersichtlich ist. Der Ethanumsatz lag bei ca. 52,5%, die Selektivität zu Ethylen bei ca. 82,5% und die Selektivität zu Essigsäure bei ca. 12%, d.h. einer Gesamtselektivität zu kommerziellen Wertprodukten von mehr als 94%.

**Tabelle 3**

| **Parameter** | **Einheit** | **Wert** |
|---|---|---|
| Eingefüllte Katalysatoraktivmasse | Kg | 2,13 |
| Reaktoreingangsdruck | Bara | 3,81 |
| Mittlere Kühlmitteltemperatur | °C | 316 |
| GHSV | h⁻¹ bzw. (Nm3/h)_{Gas}/M³_{Kat}. | 1088 |
| **Zusammensetzung Reaktionseinsatzstrom (Mol.-Verhältnis)** | | |
| Verhältnis Ethan : Sauerstoff : Wasser(dampf) = 59 : 24 : 17 | | |

Die Ergebnisse der Stabilitätsuntersuchungen sind in Figur 2 veranschaulicht, in der eine akkumulierte Betriebszeit (Time on Stream) in h auf der Horizontalachse gegenüber einer Umsetzung von Ethan auf der linken bzw. einer Selektivität zu Ethalen auf der rechten Vertikalachse, jeweils in Prozent, dargestellt sind. Von oben nach unten sind Werte für die Selektivität zu Ethylen, Selektivität zu Essigsäure, Umsetzung von Ethan, Selektivität zu Kohlenmonoxid und Selektivität zu Kohlendioxid dargestellt.

### Beispiel einer geeigneten Anlage

In Figur 3 ist eine Anlage zur Herstellung von Olefinen gemäß einer Ausführungsform der Erfindung in Form eines stark vereinfachten Anlagendiagramms veranschaulicht und insgesamt mit 1 bezeichnet. Die Anlage 1 ist dabei nur schematisch angedeutet. Dabei wird insbesondere die prinzipielle Anordnung der Reaktionszone(n) anhand eines stark vergrößerten und nicht maßstabsgerecht gezeichneten Rohrbündelreaktors 100 dargestellt. Wenngleich nachfolgend eine Anlage 1 zur ODHE beschrieben wird, eignet sich, wie erwähnt, die vorliegende Erfindung auch zum Einsatz bei der ODH höherer Kohlenwasserstoffe. In diesem Fall gelten die nachfolgenden Erläuterungen entsprechend.

Die Anlage 1 weist, wie erwähnt, einen Rohrbündelreaktor 100 auf, dem im dargestellten Beispiel ein Ethan enthaltendes, auf beliebige Weise gewonnenes Einsatzgemisch A zugeführt wird. Das Einsatzgemisch A kann beispielsweise einer nicht dargestellten Rektifikationseinheit entnommene Kohlenwasserstoffe enthalten. Das Einsatzgemisch A kann auch beispielsweise vorgewärmt und auf andere Weise aufbereitet werden. Das Einsatzgemisch A kann bereits Sauerstoff und ggf. einen Reaktionsmoderator wie Wasserdampf enthalten, entsprechende Medien können jedoch auch stromauf oder in dem Rohrbündelreaktor 100 zugegeben werden, wie nicht gesondert dargestellt. Dem Rohrbündelreaktor 100 wird ein Produktgemisch B entnommen.

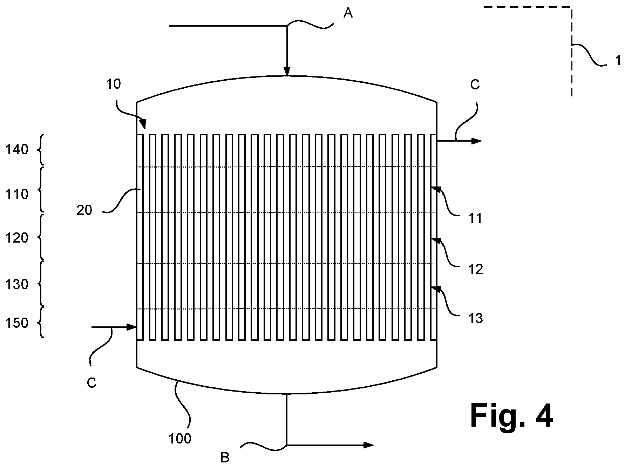

Der Reaktor 100, der in Figur 4 im Detail dargestellt ist, weist eine Vielzahl parallel angeordneter Reaktionsrohre 10 auf (nur zum Teil bezeichnet), die durch eine Vorwärmzone 140 und danach durch mehrere, im dargestellten Beispiel drei, Reaktionszonen 110, 120, 130 verlaufen. Stromab kann eine Nachreaktionszone 150 vorhanden sein. Die Reaktionsrohre 10 sind von einem Mantelbereich 20 umgeben, durch die im Beispiel ein Kühlmittel C der erläuterten Art geführt ist. Die Darstellung ist stark vereinfacht, da, wie erwähnt, die Reaktionsrohre 10 ggf. unter Verwendung mehrerer, die Reaktionsrohre 10 umfließender Kühlmedien gekühlt werden können oder unterschiedliche Rohrabschnitte unter Verwendung unterschiedlicher Kühlmedien, desselben Kühlmediums in unterschiedlichen Kühlmedienkreisläufen, und/oder desselben oder unterschiedlicher Kühlmedien in unterschiedlichen oder gleichen Strömungsrichtungen gekühlt werden können.

Nach der Einspeisung in den Rohrbündelreaktor wird das Einsatzgemisch A in geeigneter Weise auf einer Temperatur in einem ersten Temperaturbereich auf die Reaktionsrohre 10 verteilt. Die Reaktionsrohre weisen jeweils Katalysezonen 11, 12 und 13 auf, die in den Reaktionszonen 120, 130 und 140 liegen.

Eine katalytische Umsetzung erfolgt mittels der in den Reaktionsrohren 10 hintereinander angeordneten Katalysezonen 11, 12 und 13, die optional bereitgestellt werden können und dabei beispielsweise eine unterschiedliche Aktivität und/oder Selektivität aufweisen können. Die Parameter des Einsatzgemischs und der Reaktionsbedingungen wurden mehrfach erläutert.

## Patentansprüche

1. Verfahren zur Herstellung einer Zielverbindung, bei dem ein wenigstens eine Eduktverbindung enthaltendes Einsatzgemisch (A) gebildet, auf parallel angeordnete Reaktionsrohre (10) eines oder mehrerer Rohrbündelreaktoren (100) verteilt, und in den Reaktionsrohren (10) einer oxidativen katalytischen Umsetzung unterworfen wird, wobei Ethan als Einsatzverbindung verwendet wird und das oxidative katalytische Verfahren als oxidative Dehydrierung des Ethans durchgeführt wird, **dadurch gekennzeichnet, dass** dem Einsatzgemisch Wasserdampf in einer Menge zugegeben wird, die derart bemessen ist, dass ein Wasserdampfanteil des Einsatzgemischs 5 bis 95 Vol.-% beträgt, dem Einsatzgemisch Sauerstoff in Form eines zumindest 95 Vol.-% Sauerstoff enthaltenden Fluids zugegeben wird, und die oxidative katalytische Umsetzung unter Verwendung eines oder mehrerer, die Metalle Molybdän, Vanadium, Niob und optional Tellur enthaltender Katalysatoren, der oder die zumindest teilweise aus den Oxiden der Metalle hergestellt ist oder sind, durchgeführt wird, der oder die in mehreren, in einer Strömungsrichtung hintereinander angeordneten und in der Strömungsrichtung durchströmten Reaktionszonen (11, 12, 13) des einen oder der mehreren Rohrbündelreaktoren (100) bereitgestellt ist oder sind, wobei der eine oder die mehreren Katalysatoren in den Reaktionszonen (11, 12, 13) mit einer unterschiedlichen Katalysatorbeladung und/oder einer unterschiedlichen Katalysatoraktivitat pro Raumeinheit bereitgestellt ist oder sind.

2. Verfahren nach Anspruch 1, bei dem der Wasserdampfanteil des Einsatzgemischs 10 bis 50 Vol.-%, insbesondere 14 bis 35 Vol.-%, beträgt.

3. Verfahren nach einem der vorstehenden Ansprüche, bei dem das oxidative katalytische Verfahren bei einer Temperatur des oder der Katalysatoren in einem Bereich zwischen 240 und 500 °C insbesondere zwischen 280 und 450 °C, weiter insbesondere zwischen 300 und 400 °C durchgeführt wird, und/oder bei dem ein Gesamtdruck des Einsatzgemischs an einem Eintritt des oder der Rohrbündelreaktoren (100) 1 bis 10 bar (abs.), insbesondere 2 bis 6 bar (abs.) beträgt.

4. Verfahren nach einem der vorstehenden Ansprüche, bei dem die Reaktionsrohre unter Verwendung eines oder mehrerer, die Reaktionsrohre (10) umfließender Kühlmedien gekühlt werden.

5. Verfahren nach Anspruch 4, bei dem unterschiedliche Abschnitte der Reaktionsrohre (10) unter Verwendung unterschiedlicher Kühlmedien, unter Verwendung desselben Kühlmediums in unterschiedlichen Kühlmedienkreisläufen, und/oder unter Verwendung desselben oder unterschiedlicher Kühlmedien in unterschiedlichen oder gleichen Strömungsrichtungen gekühlt werden.

6. Verfahren nach einem der vorstehenden Ansprüche, bei dem ein maximaler Temperaturunterschied von 60 K, 55 K, 50 K, 45 K oder 40 K zwischen einem oder mehreren Temperaturhotspots der Reaktionszonen (11, 12, 13) und einer Kühlmitteltemperatur eingehalten wird.

7. Verfahren nach einem der vorstehenden Ansprüche, bei dem der oder die Katalysatoren unter Verwendung einer Hydrothermalsynthese, insbesondere in einem Autoklaven und insbesondere unter Verwendung eines Drucks im Bereich von 5 bis 50 bar (abs.), insbesondere von 10 bis 35 bar (abs.), weiter insbesondere von 15 bis 30 bar (abs.), und einer Temperatur im Bereich von 150 bis 280 °C, insbesondere von 150 bis 230 °C, weiter insbesondere von 170 bis 210 °C hergestellt ist.

8. Verfahren nach Anspruch 6 oder 7, bei dem der oder die Katalysatoren durch Kristallisation unter hydrothermalen Bedingungen unter Verwendung von Molybdäntrioxid, Divanadiumpentoxid und optional Tellurdioxid und unter Verwendung von Oxo-Liganden hergestellt wird, wobei die Oxo-Liganden insbesondere jeweils mindestens zwei Sauerstoffatome aufweisen und aus Carbonsäuren und Alkoholen, insbesondere aus Oxalsäure, Zitronensäure und 1,2-Alkandiolen ausgewählt sind.

9. Verfahren nach einem der Ansprüche 6 bis 8, bei dem der oder die Katalysatoren durch Verpressen in eine Partikelform gebracht ist.

10. Verfahren nach einem der Ansprüche 6 bis 9, wobei der oder die Katalysatoren insbesondere eine katalytisch inaktive Komponente, insbesondere ein katalytisch inaktives Metalloxid, aufweist, und wobei in den Reaktionsrohren Katalysezonen bereitgestellt sind, in denen der oder die Katalysatoren in unterschiedlichen Mengen mit dem inaktiven Metalloxid verdünnt sind.

## Claims

1. Process for producing a target compound, in which a feed mixture (A) containing at least one reactant compound is formed, distributed onto parallel reaction tubes (10) of one or more multi-tube reactors (100), and subjected to an oxidative catalytic reaction in the reaction tubes (10), ethane being used as the feed compound and the oxidative catalytic process being carried out as oxidative dehydrogenation of the ethane, **characterized in that** steam is added to the feed mixture in an amount such that the steam content of the feed mixture is 5 to 95 vol.%, oxygen is added to the feed mixture in the form of a fluid containing at least 95 vol.% oxygen, and the oxidative catalytic reaction is carried out using one or more catalysts which contains or contain the metals molybdenum, vanadium, niobium and optionally tellurium, is or are at least partially made from the oxides of the metals, and is or are provided in a plurality of reaction zones (11, 12, 13) of the one or more multi-tube reactors (100), which reaction zones are arranged one after the other in a flow direction and through which the fluid flows in the flow direction, the one or more catalysts in the reaction zones (11, 12, 13) being provided with a different catalyst load and/or a different catalyst activity per unit of volume.

2. Process according to claim 1, in which the steam content of the feed mixture is 10 to 50 vol.%, in particular 14 to 35 vol.%.

3. Process according to either of the preceding claims, in which the oxidative catalytic process is carried out at a temperature of the catalyst(s) in a range between 240 and 500°C, in particular between 280 and 450°C, and more in particular between 300 and 400°C, and/or in which the total pressure of the feed mixture at an inlet of the multi-tube reactor(s) (100) is 1 to 10 bar (abs.), in particular 2 to 6 bar (abs.).

4. Process according to any of the preceding claims, in which the reaction tubes are cooled using one or more cooling media which flow around the reaction tubes (10).

5. Process according to claim 4, in which different portions of the reaction tubes (10) are cooled using different cooling media, using the same cooling medium in different cooling media circuits, and/or using the same or different cooling media in different or the same flow directions.

6. Process according to any of the preceding claims, in which a maximum temperature difference of 60 K, 55 K, 50 K, 45 K or 40 K is maintained between one or more temperature hot spots of the reaction zones (11, 12, 13) and a coolant temperature.

7. Process according to any of the preceding claims, in which the catalyst(s) is/are produced using hydrothermal synthesis, in particular in an autoclave and in particular using a pressure in the range of 5 to 50 bar (abs.), in particular 10 to 35 bar (abs.), more in particular 15 to 30 bar (abs.), and using a temperature in the range of 150 to 280°C, in particular 150 to 230°C, more in particular 170 to 210°C.

8. Process according to claim 6 or 7, in which the catalyst(s) is/are produced by crystallization under hydrothermal conditions using molybdenum trioxide, divanadium pentoxide and optionally tellurium dioxide and using oxo ligands, wherein the oxo ligands in particular each have at least two oxygen atoms and are selected from carboxylic acids and alcohols, in particular from oxalic acid, citric acid and 1,2-alkanediols.

9. Process according to any of claims 6 to 8, in which the catalyst(s) is/are formed into a particle shape by compression.

10. Process according to any of claims 6 to 9, wherein the catalyst(s) in particular comprises/comprise a catalytically inactive component, in particular a catalytically inactive metal oxide, and wherein catalytic zones are provided in the reaction tubes, in which zones the catalyst(s) is/are diluted using the inactive metal oxide in different amounts.

## Revendications

1. Procédé pour la production d'un composé cible, dans lequel un mélange d'alimentation (A) contenant au moins un composé de départ est formé, réparti sur des tubes de réaction (10) disposés en parallèle d'un ou de plusieurs réacteurs à faisceau tubulaire (100), et soumis à une réaction d'oxydation catalytique dans les tubes de réaction (10), dans lequel de l'éthane est utilisé comme composé d'alimentation et le procédé d'oxydation catalytique est réalisé sous la forme d'une déshydrogénation oxydante de l'éthane, **caractérisé en ce que** de la vapeur d'eau est ajoutée au mélange d'alimentation en une quantité mesurée de telle sorte qu'une proportion de vapeur d'eau du mélange d'alimentation va de 5 à 95 % en volume, de l'oxygène est ajouté au mélange d'alimentation sous la forme d'un fluide contenant au moins 95 % en volume d'oxygène, et la réaction d'oxydation catalytique est réalisée en utilisant un ou plusieurs catalyseurs contenant les métaux molybdène, vanadium, niobium et éventuellement tellure, qui sont produits au moins partiellement à partir des oxydes des métaux et qui sont fournis dans plusieurs zones de réaction (11, 12, 13) du ou des réacteurs à faisceau tubulaire (100) disposées les unes derrière les autres dans une direction d'écoulement et traversées dans la direction d'écoulement, dans lequel le ou les catalyseurs sont fournis dans les zones de réaction (11, 12, 13) avec des charges de catalyseurs différentes et/ou des activités de catalyseurs différentes par unité d'espace.

2. Procédé selon la revendication 1, dans lequel la proportion de vapeur d'eau du mélange d'alimentation va de 10 à 50 % en volume, en particulier de 14 à 35 % en volume.

3. Procédé selon l'une des revendications précédentes, dans lequel le procédé d'oxydation catalytique est mis en oeuvre à une température du ou des catalyseurs comprise dans une plage comprise entre 240 et 500 °C, en particulier entre 280 et 450 °C, plus particulièrement entre 300 et 400 °C, et/ou dans lequel une pression totale du mélange d'alimentation à une entrée du ou des réacteurs à faisceau tubulaire (100) est comprise entre 1 et 10 bar (abs.), en particulier entre 2 et 6 bar (abs.).

4. Procédé selon l'une des revendications précédentes, dans lequel les tubes de réaction sont refroidis en utilisant un ou plusieurs milieux de refroidissement circulant autour des tubes de réaction (10).

5. Procédé selon la revendication 4, dans lequel différentes sections des tubes de réaction (10) sont refroidies en utilisant différents milieux de refroidissement, en utilisant le même milieu de refroidissement dans différents circuits de milieu de refroidissement, et/ou en utilisant le même milieu de refroidissement ou différents milieux de refroidissement dans des directions d'écoulement différentes ou identiques.

6. Procédé selon l'une des revendications précédentes, dans lequel une différence de température maximale de 60 K, 55 K, 50 K, 45 K ou 40 K est maintenue entre un ou plusieurs points chauds de température des zones de réaction (11, 12, 13) et une température de milieu de refroidissement.

7. Procédé selon l'une des revendications précédentes, dans lequel le ou les catalyseurs sont produits en utilisant une synthèse hydrothermale, en particulier dans un autoclave et en particulier en utilisant une pression comprise dans la plage allant de 5 à 50 bar (abs.), en particulier de 10 à 35 bar (abs.), plus particulièrement de 15 à 30 bar (abs.), et une température comprise dans la plage allant de 150 à 280 °C, en particulier de 150 à 230 °C, plus particulièrement de 170 à 210 °C.

8. Procédé selon la revendication 6 ou 7, dans lequel le ou les catalyseurs sont produits par cristallisation dans des conditions hydrothermales en utilisant du trioxyde de molybdène, du pentoxyde de divanadium et éventuellement du dioxyde de tellure et en utilisant des oxo-ligands, dans lequel les oxo-ligands présentent en particulier respectivement au moins deux atomes d'oxygène et sont choisis parmi acides carboxyliques et alcools, en particulier parmi acide oxalique, acide citrique et 1,2-alcanediols.

9. Procédé selon l'une des revendications 6 à 8, dans lequel le ou les catalyseurs sont mis sous forme de particules par compression.

10. Procédé selon l'une des revendications 6 à 9, dans lequel le ou les catalyseurs présentent en particulier un composant catalytiquement inactif, en particulier un oxyde métallique catalytiquement inactif, et dans lequel des zones de catalyse sont fournies dans les tubes de réaction, dans lesquelles le ou les catalyseurs sont dilués en différentes quantités avec l'oxyde métallique inactif.
